(19) Europäisches Patentamt
European Patent Office
Office européen des brevets

(11) Publication number: **0 093 805**
**B1**

(12) **EUROPEAN PATENT SPECIFICATION**

(45) Date of publication of patent specification: **13.05.87**

(21) Application number: **82300793.5**

(22) Date of filing: **17.02.82**

(51) Int. Cl.⁴: **C 07 D 209/44,**
C 07 D 209/46, A 61 K 31/40

# (54) Octahydro-2-(omega-mercaptoalkanoyl)3-oxo-1H-isoindole-1-carboxylic acids and esters.

(30) Priority: **17.02.81 US 235381**
**07.12.81 US 327651**

(43) Date of publication of application:
**16.11.83 Bulletin 83/46**

(45) Publication of the grant of the patent:
**13.05.87 Bulletin 87/20**

(84) Designated Contracting States:
**AT BE CH DE FR GB IT LI LU NL SE**

(56) References cited:
**EP-A-0 012 845**
**EP-A-0 018 104**
**EP-A-0 031 741**
**EP-A-0 052 870**
**BE-A- 608 905**
**FR-A-2 448 533**
**GB-A-2 027 025**

(73) Proprietor: **WARNER-LAMBERT COMPANY**
**201 Tabor Road**
**Morris Plains New Jersey 07950 (US)**

(72) Inventor: **Blankley, Clifton John**
**3724 Vorhies**
**Ann Arbor Michigan 48105 (US)**

(74) Representative: **Jones, Michael Raymond et al**
**HASELTINE LAKE & CO. Hazlitt House**
**28 Southampton Buildings Chancery Lane**
**London WC2A 1AT (GB)**

**EP 0 093 805 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to octahydro-2-(ω-mercaptoalkanoyl)-3-oxo-1H-isoindole-1-carboxylic acids and esters.

The invention provides octahydro-2-(ω-mercaptoalkanoyl)-3-oxo-1H-isoindole-1-carboxylic acids and esters having the formula

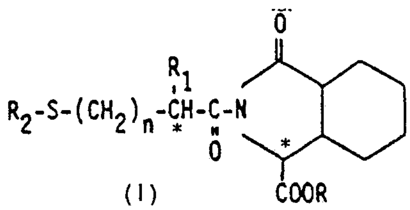

(I)

wherein R is hydrogen or lower alkyl, $R_1$ is hydrogen, lower alkyl or benzyl, $R_2$ is hydrogen or $R_3$—CO— (wherein $R_3$ is lower alkyl, furyl, benzo(b)furyl, thienyl, benzo(b)thienyl, pyridyl, quinolyl, isoquinolyl, phenyl or substituted phenyl having one or two substituents selected from fluorine, chlorine, bromine, lower alkyl and lower alkoxy), and n is 0 or 1; and pharmaceutically acceptable salts thereof when R is hydrogen and/or when $R_3$ is pyridyl, quinolyl or isoquinolyl. The terms lower alkyl and lower alkoxy include groups having straight or branched chains and containing 1 to 4 carbon atoms.

Preferred compounds of the invention are octahydro-2-(ω-mercaptoalkanoyl)-3-oxo-1H-isoindole-1-carboxylic acids having the formula

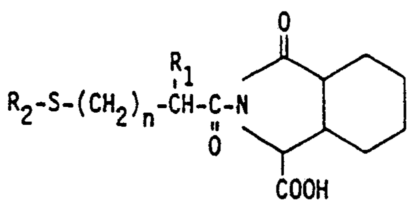

and pharmaceutically acceptable salts thereof, wherein $R_1$ is hydrogen or lower alkyl containing 1 to 3 carbon atoms, $R_2$ is hydrogen or $R_3$—CO— (wherein $R_3$ is lower alkyl containing 1 to 3 carbon atoms, phenyl, furyl, benzo(b)furyl, thienyl, benzo(b)thienyl, pyridyl, quinolyl or isoquinolyl), and n is as defined above.

Further preferred compounds of the invention are octahydro-2-(3-mercaptopropanoyl)-3-oxo-1H-isoindole-1-carboxylic acids having the formula

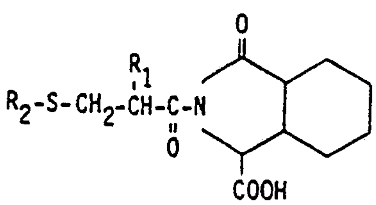

and pharmaceutically acceptable basic salts thereof, wherein $R_1$ is hydrogen or methyl, and $R_2$ is hydrogen or $R_3$—CO— (wherein $R_3$ is methyl or phenyl).

The preferred specific compounds of the invention are: octahydro-2-(3-mercaptopropanoyl)-3-oxo-1H-isoindole-1-carboxylic acid, octahydro-2-(3-mercapto-2-methylpropanoyl)-3-oxo-1H-isoindole-1-carboxylic acid, and the pharmaceutically acceptable basic salts thereof.

The compounds of the invention have asymmetric carbon atoms. These carbon atoms are indicated by an asterisk in formula I. Additional asymmetric carbon atoms may be present in the lower alkyl groups. The compounds accordingly exist as optical isomers and diastereomers or as racemates and mixtures thereof. All of these are within the scope of the invention. The *S* configuration at the centers marked with an asterisk in formula I is preferred.

The octahydro-1H-isoindole-3-oxo-1-carboxylic acid nucleus of the compounds of this invention may potentially exist in several isomeric forms. The stereochemistry at the fused ring junction is believed to be *cis*. The carboxylic acid group may be either *cis* or *trans* to the fused cyclohexane ring.

The compounds of the invention may exist in anhydrous form as well as in solvated, including hydrated, forms. In general, the hydrated forms and the solvated forms with pharmaceutically acceptable solvents are equivalent to the anhydrous or unsolvated form for the purposes of the invention.

The compounds of the invention which have the formula:

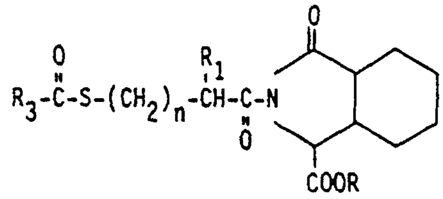

can be produced by reacting octahydro-3-oxo-1H-isoindole-1-carboxylic acid compounds of formula IV

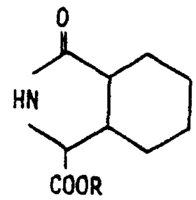

IV

with an ω-$R_3$—CO-mercaptoalkanoic acid halide of formula V

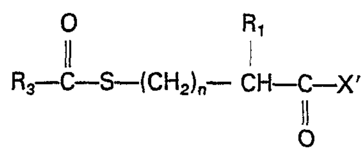

(V)

in a basic medium, where X' is halogen, preferably chlorine or bromine, and R, $R_1$, $R_3$ and n have the significance specified above. The basic media can be provided preferably by the use of an excess of a tertiary organic amine such as pyridine or triethylamine, an alkali or alkaline earth metal hydroxide, an alkali metal bicarbonate, an alkali metal carbonate or other inorganic base capable of neutralizing the hydrogen halide formed during the reaction. The reaction is carried out at a temperature of about 0°C to about 45°C under anhydrous or aqueous conditions. Suitable organic solvents for the reaction include dichloromethane, tetrahydrofuran, dioxane, chloroform, pyridine, and triethylamine. The reaction is quite rapid and is usually complete in about one-half to four hours.

The compounds of the invention wherein $R_2$ is an R—CO— group and R is hydrogen can, in accordance with the invention, also be produced by reacting a trimethylsilyl ester of octahydro-3-oxo-1H-isoindole-1-carboxylic acid IV with an ω-$R_3$—CO-mercaptoalkanoic acid halide V followed by hydrolysis of the intermediate trimethylsilyl ester compound to the free acid by treatment with water. The first step of the process can be carried out in a non-protic solvent such as methylene chloride, tetrahydrofuran, dioxane, chloroform or acetonitrile at an elevated temperature, usually about 60°C to 100°C. After the reaction is complete, about one-half to one hour, the intermediate trimethylsilyl ester compound can be treated with water at room temperature to produce the desired product.

The compounds of the invention wherein both R and $R_2$ are hydrogen which have the formula

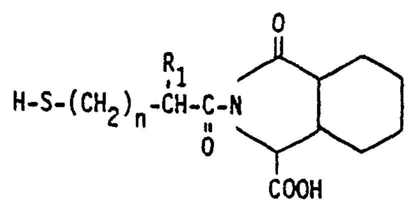

can, in accordance with the invention, be produced by hydrolyzing a compound of the invention which has the formula

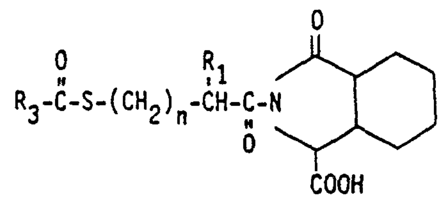

wherein R, $R_1$, $R_3$ and n have the same significance as given above. The hydrolysis is most conveniently carried out by reacting said compound with an excess of an alklai metal hydroxide in an aqueous alcoholic solution under an inert atmosphere for 5 minutes to 24 hours at a temperature of about 20°C to about 80°C.

The products wherein R and $R_2$ are both hydrogen can also be produced by ammonolysis of a compound of formula

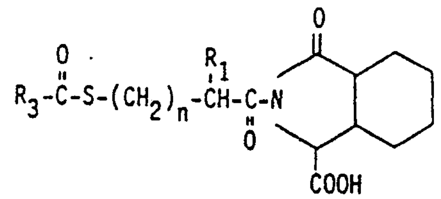

wherein $R_1$, $R_3$ and n have the same significance as given above. The ammonolysis is most conveniently carried out at room temperature in an alcohol which has been saturated with gaseous ammonia. The reaction usually requires 1 to 24 hours for completion.

The above described synthesis can utilize the racemate or one of the enantiomers as starting material. When the racemic starting material is used in the synthetic procedure, the diastereomers obtained in the product can be separated by conventional chromatographic or fractional crystallization methods.

Compounds of the invention of formula I where $R_2$ is $R_3$—CO— may alternately be prepared from compounds where $R_2$ is hydrogen by tratment of the latter with a suitable acylating agent $R_3$—CO—X″ wherein X″ is a leaving group, e.g. Cl, Br, O—CO—$R_3$, or the group:

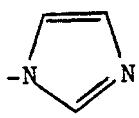

in the presence of a base, e.g. alkali carbonates or tertiary organic amines, or in aprotic solvents, e.g. dimethylformamide, tetrahydrofuran or chlorinated hydrocarbons. These may be purified as the free acids or isolated as salts with hindered organic amines, e.g. dicyclohexylamine or t-butylamine.

The products are obtained typically as a mixture of disastereomers which can be separated by standard methods of fractional crystallization or chromatography.

The compounds of this invention form basic salts with various inorganic and organic bases which are also within the scope of the invention. Such salts include ammonium salts, alkali metal salts like sodium and potassium salts, alkaline earth metal salts like the calcium and magnesium salts, salts with organic bases, e.g. dicyclohexylamine or benzathine, salts with basic amino acids like arginine, lysine and the like. The pharmaceutically acceptable salts are preferred, although other salts such as the dicyclohexylamine salt are also useful, e.g. in isolating, purifying or characterizing the product.

The salts are formed in conventional manner by reacting the free acid form of the product with one or more equivalents of the appropriate base providing the desired cation in a solvent or medium in which the salt is insoluble, or in water and removing the water by freeze drying.

In the compounds of formula I when $R_3$ is heteroaryl containing 1 or 2 nitrogen atoms the pharmaceutically acceptable acid addition salts may be prepared by conventional reactions with equivalent amounts of organic or inorganic acids. As exemplary, but not limiting, of pharmaceutically acceptable acid salts are the salts of hydrochloric, sulfuric, acetic, fumaric, malic, maleic and citric acids.

The action of the enzyme renin on angiotensinogen, a pseudoglobulin in blood plasma, produces the decapeptide angiotensin I. Angiotensin I is converted by angiotensin converting enzyme (ACE) to the octapeptide angiotensin II. The latter is an active pressor substance which has been implicated as the causative agent in various forms of hypertension in various mammalian species, e.g., rats and dogs. The compounds of this invention intervene in the renin->angiotensin I-> angiotensin II sequence by inhibiting angiotensin I converting enzyme and reducing or eliminating the formation of the pressor substance angiotensin II, and therefore are useful in reducing or relieving hypertension. Thus by the administration of a composition containing one or a combination of compounds of formula I or a pharmaceutically acceptable salt thereof, hypertension in the species of mammal suffering therefrom is alleviated. A single dose, or preferably two to four divided daily doses, provided on a basis of about 0.1 to 100 mg per kilogram per day, preferably about 1 to 50 mg per kilogram per day is appropriate to reduce blood pressure. The substance is preferably administered orally, but parenteral routes such as subcutaneously, intramuscularly, intravenously or intraperitonealy can also be employed.

The following Table shows the *in vitro* activity of compounds of formula VI in an assay for angiotensin converting enzyme inhibitory activity which is a modification of a test reported by D. Cushman and H. Cheung, Biochemical Pharmacology, *20*, 1637—1648 (1971).

*In vitro ACE Assay*: Angiotensin converting enzyme (ACE) inhibitory activity is determined by assaying guinea pig serum ACE in the presence and absence of the test compound. ACE from guinea pig serum and the test compounds are preincubated for 10 minutes before the addition of the labelled substrate $^3$H-hippuryl-glycylglycine. After a 60 minutes incubation at 37°C the reaction is stopped by the addition of 0.1N HCl. ACE cleaves the hippuryl-glycol bond to form the dipeptide glycyl-glycine and $^3$H-hippuric acid. The $^3$H-hippuric acid is then extracted with ethyl acetate and the ACE inhibition of a given sample calculated on the basis of the $^3$H-hippuric acid generated.

TABLE
Activity of Compounds of Formula VI

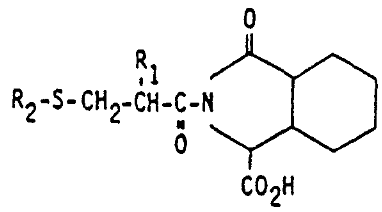

VI

| $R_1$ | $R_2$ | $IC_{50}(\mu M)$ |
|---|---|---|
| H | $COCH_3$ | 0.66 |
| H | H | 0.013 |
| $CH_3$ | $COCH_3$ | 0.28 |
| $CH_3$ | H | 0.014 |

The $IC_{50}$ is the molar concentration of compound which inhibits 50% of the conversion of angiotensin *I* to angiotension *II*.

The compounds of the invention can be utilized to reduce blood pressure in the form of tablets, capsules or elixirs for oral administration or in sterile solutions or suspensions for parenteral administration. About 10 to 500 mg. of a compound or mixture of compounds of formula I or a pharmaceutically acceptable salt is compounded with a pharmaceutically acceptable vehicle or carrier which may contain excipients, binders, preservatives, stabilizers, flavors, etc., in accord with accepted pharmaceutical practice. The amount of active substance in these compositions or preparations is such that a suitable dosage in the range indicated is obtained.

Illustrative of the inert ingredients which may be incorporated in tablets, capsules and the like are the following: A binder such as gum tragacanth, acacia, corn starch or gelatin; an excipient such as dicalcium phosphate; a disintegrating agent such as corn starch, potato starch, alginic acid and the like; a lubricant such as magnesium stearate; a sweetening agent such as sucrose, lactose or saccharin; a flavouring agent such as peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc., or a synthetic fatty vehicle like ethyl oleate or the like. Buffers, preservatives, antioxidants and the like can be incorporated as required.

The invention is illustrated by the following Examples 5 to 8. Examples 1 to 4 illustrate, for comparative purposes, the production of compounds in which the 3-oxo atom is replaced by two hydrogen atoms; as well as the formation of certain salts of these compounds; and Examples 9 to 11 illustrate the production of typical pharmaceutical formulations of the 3-(2H) compounds, it being appreciated that formulations of the corresponding 3-oxo- compounds can be prepared in a similar manner.

Example 1

2-[3-(Acetylthio)-1-oxopropyl]octahydro-1H-isoindole-1-carboxylic acid.

A mixture of 2.0 g of octahydro-1H-isoindole-1-carboxylic acid hydrochloride, 2.3 g of pyridine and 25 ml of tetrahydrofuran is cooled to 0°C and treated dropwise with 3-(acetylthio)-propanoyl chloride. The mixture is stirred for 2 hours at 0°C and allowed to warm to room temperature during another hour. The solvent is removed at reduced pressure. The residue is treated with water, acidified with 6N sulfuric acid and extracted with ethyl acetate. Drying, filtration, and concentration of the organic layer gives the crude product as an oil which solidifies. A pure sample is obtained after recrystallization from ethyl acetate and has mp 140—145°C.

Octahydro-1H-isoindole-1-carboxylic acid hydrochloride used as an intermediate in this preparation is obtained by the following method. Following a procedure given in Gazz. Chim. Ital, *106*, 65 (1976), 2,3-dihydro-1H-isoindole-1-carboxylic acid, methyl ester, hydrochloride is prepared. This compound, 2.14 g, is

dissolved in 100 ml of methanol and 5 ml of acetic acid and hydrogenated over 0.5 g of 10% rhodium/carbon catalyst. The catalyst is removed by filtration and the filtrate is concentrated to give a residue which solidifies on trituration with ether. Recrystallization from methanol-ether gives a pure sample of methyl octahydro-1H-isoindole-1-carboxylate hydrochloride, mp 181—183°C.

This compound, 6.25 g, is combined with 17.5 ml of concentrated hydrochloric acid and 36 ml of water and heated at reflux for 4 hours. The solvent is removed at reduced pressure and the wet residue is recrystallized from water to give octahydro-1H-isoindole-1-carboxylic acid, hydrochloride, mp 257—262°C (dec).

Example 2

2-(3-Mercaptopropanoyl)octahydro-1H-isoindole-1-carboxylic acid, dicyclohexylamine salt.

A solution of 1.2 g of 2-[3-(acetylthio)-1-oxopropyl]octahydro-1H-isoindole-1-carboxylic acid in 10 ml of 5N ammonia in methanol is prepared under nitrogen and allowed to stand for 2 hours. The solvent is removed at reduced pressure and the resulting residue is treated with 10% potassium bisulfate solution and extracted with ethyl acetate. The organic layer is dried and concentrated to give the crude product. This is purified as the dicyclohexylamine salt which is recrystallized from ethyl acetate, has mp 170—175°C (dec) and contains varying amounts of water of hydration.

Salts

*Sodium*

Octahydro-2-(3-mercapto-2-methylpropanoyl)-1H-isoindole-1-carboxylic acid (5 mg) is dissovled in a solution of water (2.5 ml) and an equivalent amount of 1*N* sodium hydroxide. The solution is freeze dried to obtain the sodium salt.

*Magnesium*

Octahydro-2-(3-mercaptopropanoyl)-1H-isoindole-1-carboxylic acid (5 mg) magnesium oxide (49.5 mg) and water (10 ml) are stirred with slight heating until complete solution is obtained. Then the solvent is removed by freeze drying to obtain the magnesium salt.

*Calcium*

Octahydro-2-(3-mercaptopropanoyl)-1H-isoindole-1-carboxylic acid (5 mg) is dissolved in a mixture of calcium hydroxide (91 mg) and water (10 ml), and the solution is freeze dried to obtain the calcium salt.

*Potassium*

Octahydro-2-(3-mercaptopropanoyl)-1H-isoindole-1-carboxylic acid (5 mg) is dissolved in a mixture of an equivalent amount of potassium bicarbonate and water (10 ml) and freeze dried to obtain the potassium salt.

Example 3

2-[3-(Acetylthio)-2-methyl-1-oxopropyl]octahydro-1H-isoindole-1-carboxylic acid.

Following the procedure of Example 1 but using 3-(acetylthio)-2-methylpropanoyl chloride in place of 3-(acetylthio)propanoyl chloride, 2-[3-(acetylthio)-2-methyl-1-oxopropyl]octahydro-1H-isoindole-1-carboxylic acid is prepared. This oil has an $R_f$ of 0.54 (silica gel; 1:1 chloroform/methanol).

Example 4

2-[3-Mercapto-2-methyl-1-oxopropyl]octahydro-1H-isoindole-1-carboxylic acid.

Following the procedure of Example 2, 2-[3-(acetylthio)-2-methyl-1-oxopropyl]octahydro-1H-isoindole-1-carboxylic acid is converted to 2-[3-mercapto-2-methyl-1-oxopropyl]octahydro-1H-isoindole-1-carboxylic acid, mp 119—129°C as the free acid; $R_f$ = 0.50 (silica gel; 1:1 chloroform/methanol).

Example 5

2-[3-Acetylthio-1-oxopropyl]octahydro-3-oxo-1H-isoindole-1-carboxylic acid, dicyclohexylamine salt.

Octahydro-2-oxo-1H-isoindole-1-carboxylic acid, 1.5 g, in 20 ml of acetonitrile is stirred while 1.4 g of hexamethyldisilazane is added. A few drops of chlorotrimethylsilane is added as catalyst and the mixture is heated at reflux with protection from moisture for 5 hours. After removal of the solvent, the residue is dissolved in 20 ml of toluene and treated dropwise with 3-(acetylthio)propanoyl chloride. The solution is distilled slowly under partial reflux until the head temperature reaches 85°C. The remaining solvent is then removed under reduced pressure and the residual oil is partitioned between ethyl acetate and water. The organic layer is dried and concentrated at reduced pressure to give crude product. This may be purified by chromatography over silica gel, eluting with a 95:5:1 mixture of chloroform/methanol/acetic acid. The product is further purified as its dicyclohexylamine salt, which has mp 198—203°C after recrystallization from acetonitrile. The product may contain varying amounts of water of hydration.

The intermediate octahydro-3-oxo-1H-isoindole-1-carboxylic acid is prepared as follows. 2,3-Dihydro-3-oxo-1H-isoindole-1-carboxylic acid (J. Prakt, Chem., *146*, 307 (1936)), 20 g is heated at reflux for 2 hours with 250 ml of absolute ethanol and 5.6 g of concentrated sulfuric acid. On cooling, the solution deposits crystalline ethyl 2,3-dihydro-3-oxo-1H-isoindole-3-carboxylate. Recrystallization of this from ethanol gives pure material, mp 178—183°C.

This ester, 15 g, is dissolved in 110 ml of tetrahydrofuran and 110 ml of absolute ethanol, treated with 1.0 g of 10% Rh/C catalyst and hydrogenated at an initial pressure of 50 psi and 36°C. After the required

amount of hydrogen has been absorbed, the mixture is filtered and the filtrate is concentrated to give the crude product. Pure ethyl octahydro-3-oxo-1H-isoindole-1-carboxylate has mp 149—157°C after recrystallization from ethanol.

This ester, 5 g, is hydrolyzed by treating it with a mixture of 48 ml 1N sodium hydroxide and 10 ml of ethanol overnight at room temperature. Partial concentration to remove ethanol and acidification precipitates the crude acid. Octahydro-3-oxo-1H-isoindole-1-carboxylic acid has mp 205—213°C (decomposition) after recrystallization from water.

Example 6

2-[3-Mercapto-1-oxopropyl]octahydro-3-oxo-1H-isoindole-1-carboxylic acid, dicyclohexylamine salt.

A mixture of 1.6 g of 2-[3-(acetylthio)-1-oxopropyl]octahydro-3-oxo-1H-isoindole-1-carboxylic acid, dicyclohexylamine salt and a solution of 1.8 g of potassium bisulfate in 80 ml of water is stirred for 10 minutes. The free carboxylic acid is extracted into ethyl acetate and the organic layer is dried and concentrated under reduced pressure. The residue is then hydrolyzed following the procedure of Example 2. The product is isolated as a dicyclohexylamine salt, mp 188—208°C after recrystallization from acetonitrile.

Example 7

2-[3-(Acetylthio)-2-methyl-1-oxopropyl]octahydro-3-oxo-1H-isoindole-1-carboxylic acid, dicyclohexylamine salt.

This compound is prepared following the procedure of Example 5 using 3-acetylthio-2-methylpropanoyl chloride as intermediate. The product is purified by recrystallization from ethyl acetate and has mp 185—188°C.

Example 8

2-[3-Mercapto-2-methyl-1-oxopropyl]octahydro-3-oxo-1H-isoindole-1-carboxylic acid, dicyclohexylamine salt.

This compound is prepared from 2-[3-(acetylthio)-2-methyl-1-oxopropyl]octahydro-3-oxo-1H-isoindole-1-carboxylic acid following the procedure of Example 2. The dicyclohexylamine salt is prepared and has mp 191—200°C after recrystallization from acetonitrile.

Example 9

1000 tablets each containing 100 mg of octahydro-2-(3-mercaptopropanoyl)-1H-isoindole-1-carboxylic acid are produced from the following ingredients.

| | |
|---|---|
| Octahydro-2-(3-mercaptopropanoyl)-1H-isoindole-1-carboxylic acid | 100 g |
| Corn Starch | 50 g |
| Gelatin | 7.5 g |
| Avicel (microcrystalline cellulose) | 25 g |
| Magnesium Stearate | 2.5 g |

The octahydro-2-(3-mercaptopropanoyl)-1H-isoindole-1-carboxylic acid and corn starch are admixed with an aqueous solution of the gelatin. The mixture is dried and ground to fine powder. The Avicel and then the magnesium stearate are admixed with the granulation. This is then compressed in a tablet press to form 1000 tablets each containing 100 mg of active ingredients.

Example 10

Two piece No. 1 gelatin capsules each containing 250 mg of octahydro-2-(3-mercaptopropanoyl)-1H-isoindole-1-carboxylic acid are filled with a mixture of the following ingredients.

| | |
|---|---|
| Octahydro-2-(3-mercaptopropanoyl)-1H-isoindole-1-carboxylic acid, | 250 mg |
| Magnesium Stearate | 7 mg |
| USP Lactose | 193 mg |

Example 11

An injectable solution is produced as follows:

| | |
|---|---|
| Octahydro-2-(3-mercaptopropanoyl)-1H-isoindole-1-carboxylic acid, sodium salt | 500 g |
| Methyl Paraben | 5 g |
| Propyl Paraben | 1 g |
| Sodium Chloride | 25 g |
| Water for Injection g.s. | 5 g |

The active substance, preservatives and sodium chloride are dissolved in 3 liters of water for injection and then the volume is brought up to 5 liters. The solution is filtered through a sterile filter and aseptically filled into presterilized vials which are then closed with presterilized rubber closures. Each vial contains 5 ml of solution in a concentration of 100 mg of active ingredient per ml of solution for injection.

**Claims for the Contracting States BE CH DE FR GB IT LI LU NL SE**

1. An octahydro-2-(ω-mercaptoalkanoyl)-3-oxo-1H-isoindole-1-carboxylic acid or ester having the formula

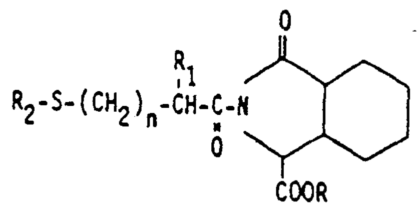

wherein R is hydrogen or a lower alkyl, $R_1$ is hydrogen, lower alkyl or benzyl, $R_2$ is hydrogen or $R_3$—CO— (wherein $R_3$ is lower alkyl, furyl, benzo(b)furyl, thienyl, benzo(b)thienyl, pyridyl, quinolyl, isoquinolyl, phenyl or substituted phenyl having one or two substituents selected from fluorine, chlorine, bromine, lower alkyl and lower alkoxy), and n is 0 or 1, and wherein lower alkyl and lower alkoxy include straight or branched groups containing 1 to 4 carbon atoms, or a pharmaceutically acceptable salt thereof when R is hydrogen and/or when $R_3$ is pyridyl, quinolyl or isoquinolyl.

2. An acid according to claim 1, having the formula

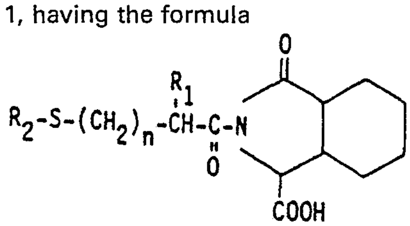

wherein $R_1$ is hydrogen or lower alkyl containing 1 to 3 carbon atoms, $R_2$ is hydrogen or $R_3$—CO— (wherein $R_3$ is lower alkyl containing 1 to 3 carbon atoms, phenyl, furyl, benzo(b) furyl, thienyl, benzo(b)thienyl, pyridyl, quinolyl or isoquinolyl), and n is 0 or 1, or a pharmaceutically acceptable salt thereof.

3. An acid according to claim 2, wherein $R_1$ is hydrogen or methyl and $R_2$ is hydrogen or $R_3$—CO— (wherein $R_3$ is methyl or phenyl), or a pharmaceutically acceptable basic salt thereof.

4. Octahydro-2-(3-mercaptopropanoyl)-3-oxo-1H-isoindole-1-carboxylic acid or a pharmaceutically acceptable salt thereof.

5. Octahydro-2-(3-mercapto-2-methylpropanoyl)-3-oxo-1H-isoindole-1-carboxylic acid or a pharmaceutically acceptable salt thereof.

6. A process for the production of an octahydro-2-(ω-mercaptoalkanoyl)-1H-isoindole-1-carboxylic acid according to claim 1, which comprises acylating a lower alkyl ester or trimethylsilyl ester of an octahydro-1H-isoindole-1-carboxylic acid with an acid halide of the formula

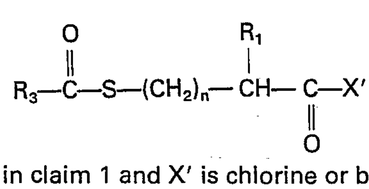

wherein $R_1$, $R_3$ and n are as defined in claim 1 and X′ is chlorine or bromine, and hydrolyzing with base to obtain the acid according to claim 1 wherein R and $R_2$ are hydrogen.

7. A pharmaceutical composition comprising an octahydro-2-(ω-mercaptoalkanoyl)-1H-isoindole-1-carboxylic acid or ester according to any of claims 1 to 5, or a pharmaceutically acceptable salt thereof according to any of claims 1 to 5, and a pharmaceutically acceptable carrier.

**Claims for the Contracting State AT**

1. A process for producing an octahydro-2-(ω-mercaptoalkanoyl)-3-oxo-1H-isoindole-1-carboxylic acid or ester having the formula

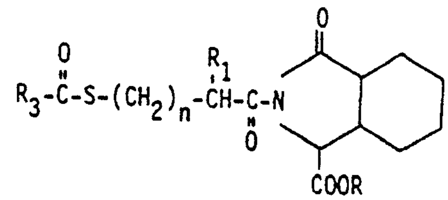

wherein R is hydrogen or a lower alkyl, $R_1$ is hydrogen, lower alkyl or benzyl, $R_3$ is lower alkyl, furyl, benzo(b)furyl, thienyl, benzo(b)thienyl, pyridyl, quinolyl, isoquinolyl, phenyl or substituted phenyl having one or two substituents selected from fluorine, chlorine, bromine, lower alkyl and lower alkoxy, and n is 0 or 1, and wherein lower alkyl and lower alkoxy include straight or branched groups containing 1 to 4 carbon atoms; which process comprises reacting an octahydro-1H-isoindole-1-carboxylic acid or ester having the formula

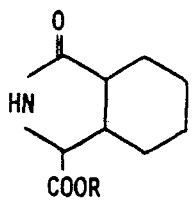

wherein R is as previously defined, with an acid halide having the formula

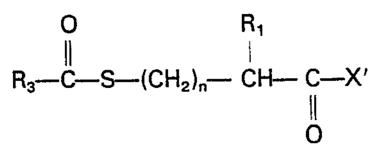

wherein X' is halogen and $R_1$, $R_3$ and n are as previously defined, in a basic medium.

2. A process for producing an octahydro-2-(ω-mercaptoalkanoyl)-3-oxo-1H-isoindole-1-carboxylic acid having the formula

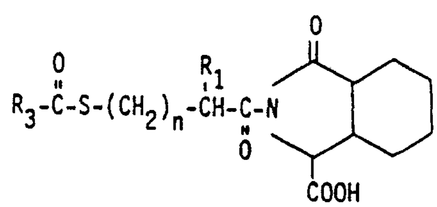

wherein $R_1$, $R_3$ and n are as defined in claim 1, which process comprises (1) reacting a trialkylsilyl ester of octahydro-1H-isoindole-1-carboxylic acid with an acid halide having the formula

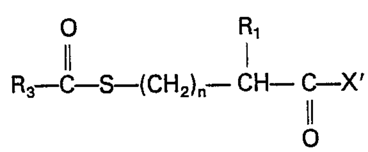

wherein $R_1$, $R_3$, n and X' are as defined in claim 1, and (2) hydrolysing the intermediate trialkylsilyl ester produced to form the free acid.

3. A process for producing an octahydro-2-(ω-mercaptoalkanoyl)-3-oxo-1H-isoindole-1-carboxylic acid having the formula

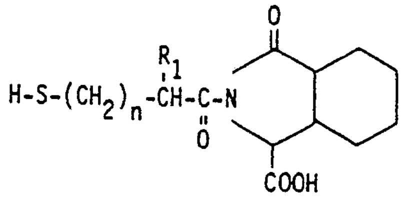

wherein $R_1$, n and X are as defined in claim 1, which process comprises hydrolysing an octahydro-2-(ω-mercaptoalkanoyl)-3-oxo-1H-isoindole-1-carboxylic acid or ester having the formula

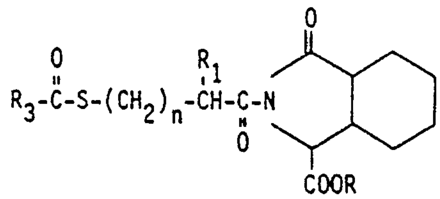

wherein R, $R_1$, $R_3$ and n are as defined in claim 1.

4. A process for producing an octahydro-2-(ω-mercaptoalkanoyl)-3-oxo-1H-isoindole-1-carboxylic acid having the formula

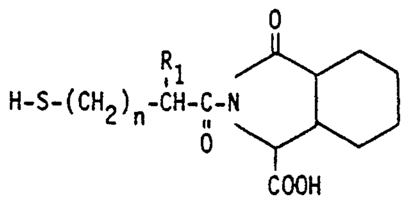

wherein $R_1$ and n are as defined in claim 1, which process comprises subjecting to ammonolysis an octahydro-2-(ω-mercaptoalkanoyl)-3-oxo-1H-isoindole-1-carboxylic acid or ester having the formula

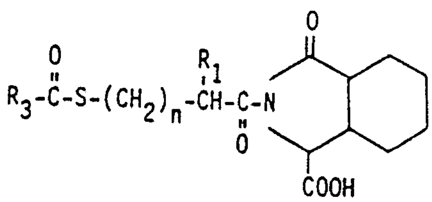

wherein $R_1$, $R_3$ and n are as defined in claim 1.

5. A process for producing an octahydro-2-(ω-mercaptoalkanoyl)-3-oxo-1H-isoindole-1-carboxylic acid or ester having the formula

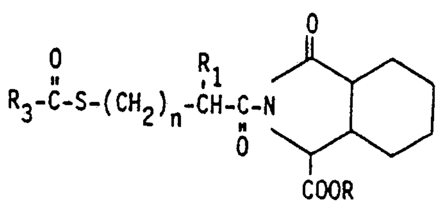

wherein R, $R_1$, $R_3$ and n are as defined in claim 1, which process comprises reacting an octahydro-2-(ω-mercaptoalkanoyl)-3-oxo-1H-isoindole-1-carboxylic acid or ester having the formula

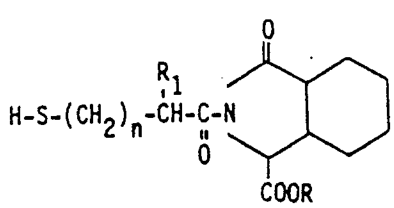

wherein R, $R_1$ and n are as defined in claim 1, with an acylating agent having the formula

$$R_3—CO—X'$$

wherein $R_3$ is as defined in claim 1 and X' is a leaving group, in the presence of a base.

6. A process for producing a pharmaceutically acceptable salt of a compound produced by a process according to any of claims 1 to 5, which process comprises reacting the compound with a base or acid capable of forming a pharmaceutically acceptable salt.

7. A process according to any of claims 1 to 6, being a process for producing an acid having the formula

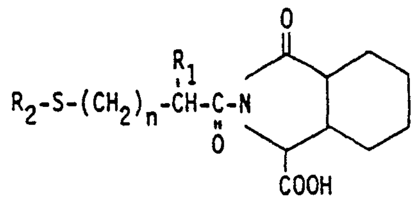

wherein $R_1$ is hydrogen or lower alkyl containing 1 to 3 carbon atoms, $R_2$ is hydrogen or $R_3$—CO— (wherein $R_3$ is lower alkyl containing 1 to 3 carbon atoms, phenyl, furyl, benzo(b)furyl, thienyl, benzo(b)thienyl, pyridyl, quinolyl or isoquinolyl), and n is 0 or 1, or a pharmaceutically acceptable salt thereof.

8. A process according to claim 7, being a process for producing an acid wherein $R_1$ is hydrogen or methyl, and $R_2$ is hydrogen or $R_3$—CO— (wherein $R_3$ is methyl or phenyl), or a pharmaceutically acceptable basic salt thereof.

9. A process according to any of claims 1 to 6, being a process for producing octahydro-2-(3-mercapto-propanoyl)-3-oxo-1H-isoindole-1-carboxylic acid or a pharmaceutically acceptable salt thereof; or octahydro-2-(3-mercapto-2-methylpropanoyl)-3-oxo-1H-isoindole-1-carboxylic acid or a pharmaceutically acceptable salt thereof.

**Revendications pour les Etats contractants: BE CH DE FR GB IT LI LU NL SE**

1. Un acide octahydro-2-(ω-mercaptoalcanoyl)-3-oxo-1H-isoindole-1-carboxylique ou ester de cet acide de formule:

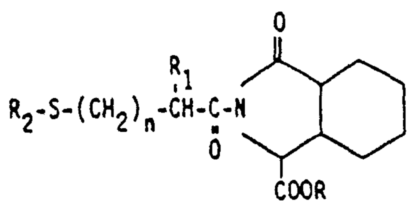

dans laquelle:

R représente un atome d'hydrogène ou un radical alkyle inférieur;

$R_1$ représente un atome d'hydrogène, un radical alkyle inférieur ou benzyle;

$R_2$ représente un atome d'hydrogène ou $R_3$—C— dans lequel $R_3$ représente un radical alkyle inférieur, furyle, benzo(b)furyle, thiényle, benzo(b)thiényle, pyridyle, quinolyle, isoquinolyle, phényle ou phényle substitué renfermant un ou plusieurs substituants choisis parmi le fluor, le chlore, le brome, un radical alkyle inférieur ou alkoxy inférieur; et

n est égal à 0 ou 1; et où par radical alkyle inférieur et alkoxy inférieur, on entend les radicaux droits ou ramifiés contenant de 1 à 4 atomes de carbone, ou un de leurs sels pharmaceutiquement acceptables dans lesquels R représente un atome d'hydrogène et/ou $R_3$ représente un radical pyridyle, quinolyle ou isoquinolyle.

2. Acide selon la revendication 1, de formule:

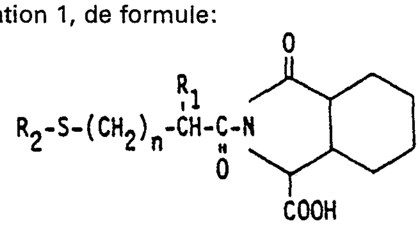

dans laquelle:

$R_1$ représente un atome d'hydrogène, ou un radical alkyle inférieur contenant de 1 à 3 atomes de carbone;

$R_2$ représente un atome d'hydrogène ou $R_3$—CO— (dans lequel $R_3$ représente un radical alkyle inférieur contenant de 1 à 3 atomes de carbone, phényle, furyle, benzo(b)furyle, thiényle, benzo(b)thiényle, pyridyle, quinolyle, isoquinolyle); et

n est égal à 0 ou 1, ou un de leurs sels pharmaceutiquement acceptables.

3. Acide selon la revendication 2, dans lequel $R_1$ représente un atome d'hydrogène ou un radical méthyle et $R_2$ représente un atome d'hydrogène ou $R_3$—CO— (dans lequel $R_3$ représente un radical méthyle ou phényle) ou un de leurs sels basiques pharmaceutiquement acceptables.

4. Acide octahydro-2-(3-mercaptopropanoyl)-3-oxo-1H-isoindole-1-carboxylique ou un de ses sels pharmaceutiquement acceptables.

5. Acide octahydro-2-(3-mercapto-2-méthylpropanoyl)-3-oxo-1H-isoindole-1-carboxylique ou un de ses sels pharmaceutiquement acceptables.

6. Un procédé de préparation d'un acide octahydro-2-(ω-mercaptoalcanoyl)-1H-isoindole-1-carboxylique selon la revendication 1, qui consiste à acyler un ester d'alkyle inférieur ou un triméthylsilylester d'un acide octahydro-1H-isoindole-1-carboxylique par un halogénure d'acide de formule:

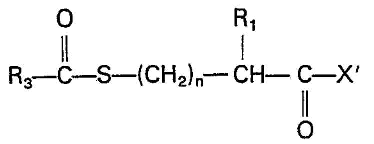

dans laquelle:

$R_1$, $R_3$ et n sont tels que définis dans la revendication 1; et

X' représente un atome de chlore ou de brome, et à hydrolyser avec une base pour obtenir l'acide selon la revendication 1 dans lequel R et $R_2$ représentent un atome d'hydrogène.

7. Une composition pharmaceutique comprenant un acide octahydro-2-(ω-mercaptoalcanoyl)-1H-isoindole-1-carboxylique ou un ester de cet acide selon une quelconque des revendications 1 à 5, ou un de leurs sels pharmaceutiquement acceptables selon une quelconque des revendications 1 à 5, et un véhicule pharmaceutiquement acceptable.

**Revendications pour l'Etat contractant: AT**

1. Un procédé de préparation d'un acide octahydro-2-(ω-mercaptoalcanoyl)-3-oxo-1H-isoindole-1-carboxylique ou ester d'un tel acide de formule:

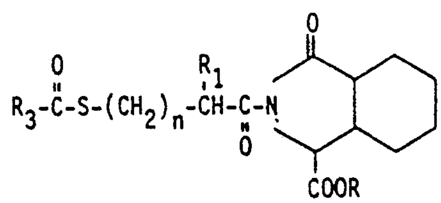

dans laquelle:

R représente un atome d'hydrogène ou un radical alkyle inférieur;

$R_1$ représente un atome d'hydrogène, un radical alkyle inférieur ou benzyle;

$R_3$ représente un radical alkyle inférieur, furyle, benzo(b)furyle, thiényle, benzo(b)thiényle, pyridyle, quinolyle, isoquinolyle, phényle ou phényle substitué renfermant un ou plusieurs substituants choisis parmi le fluor, le chlore, le brome, un radical alkyle inférieur ou alkoxy inférieur; et

n est égal à 0 ou 1; où par radical alkyle inférieur et alkoxy inférieur, on entend les radicaux droits ou ramifiés contenant de 1 à 4 atomes de carbone, ce procédé comprenant la réaction d'un acide octahydro-1H-isoindole-1-carboxylique ou ester d'un tel acide de formule:

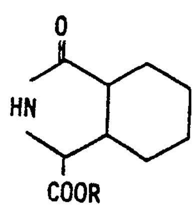

dans laquelle R est tel que défini précédemment, avec un halogénure d'acide de formule:

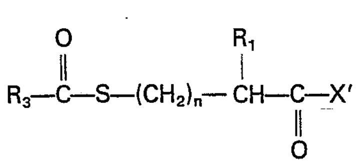

dans laquelle:

X' représente un halogène; et

$R_1$, $R_3$ et n sont tels que définis précédemment, dans un milieu basique.

2. Un procédé de préparation d'un acide ocathydro-2-(ω-mercapto-alcanoyl)-3-oxo-1H-isoindole-1-carboxylique de formule:

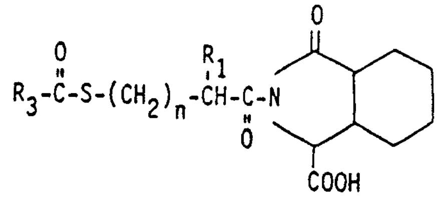

dans laquelle $R_1$, $R_3$ et n sont tels que définis dans la revendication 1, ce procédé comprenant:

(1) la réaction de l'ester trialkylsilyle de l'acide octahydro-1H-isoindole-1-carboxylique avec un halogénure d'acide de formule:

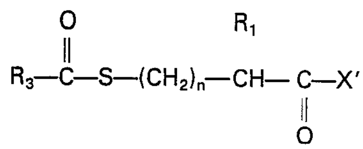

dans laquelle

$R_1$, $R_3$, n et X' sont tel que définis dans la revendication 1, et

(2) l'hydrolyse de l'ester trialkylsilyle, intermédiaire produit pour former l'acide libre.

3. Un procédé de préparation d'un acide octahydro-2-(ω-mercapto-alcanoyl)-3-oxo-1H-isoindole-1-carboxylique de formule:

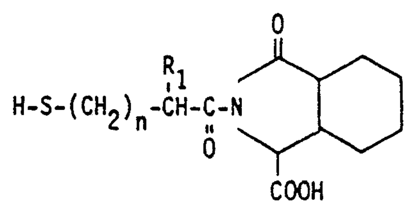

dans laquelle $R_1$, n et X sont tels que définis dans la revendication 1, lequel procédé comprend l'hydrolyse d'un acide octahydro-2-(ω-mercapto-alcanoyl)-3-oxo-1H-isoindole-1-carboxylique ou ester d'un tel acide de formule:

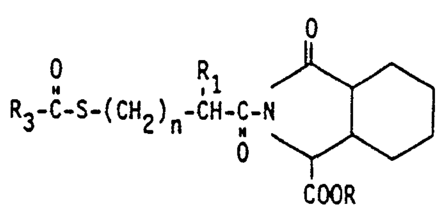

dans laquelle R, $R_1$, $R_3$ et n sont tels que définis dans la revendication 1.

4. Un procédé de préparation d'un acide octahydro-2-(ω-mercapto-alcanoyl)-3-oxo-1H-isoindole-1-carboxylique de formule:

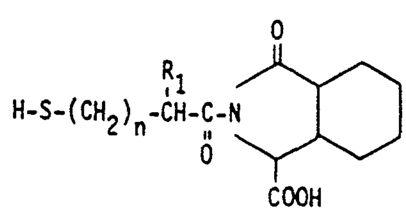

dans laquelle $R_1$ et n sont tels que définis dans la revendication 1, lequel procédé comprend l'ammonolyse d'un acide octahydro-2-(ω-mercapto-alcanoyl)-3-oxo-1H-isoindole-1-carboxylique ou ester d'un tel acide de formule:

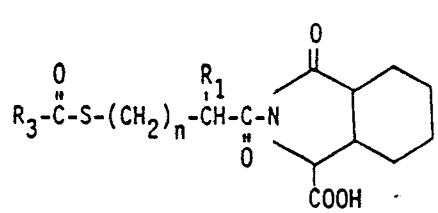

dans laquelle $R_1$, $R_3$ et n sont tels que définis dans la revendication 1.

5. Un procédé de préparation d'un acide octahydro-2-(ω-mercapto-alcanoyl)-3-oxo-1H-isoindole-1-carboxylique de formule:

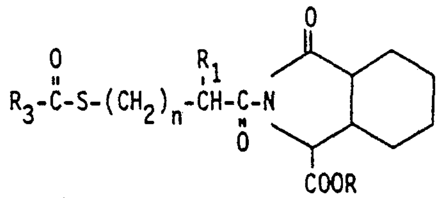

dans laquelle R, $R_1$, $R_3$ et n sont tels que définis dans la revendication 1, lequel procédé comprend la réaction d'un acide octahydro-2-(ω-mercapto-alcanoyl)-3-oxo-1H-isoindole-1-carboxylique ou ester d'un tel acide de formule:

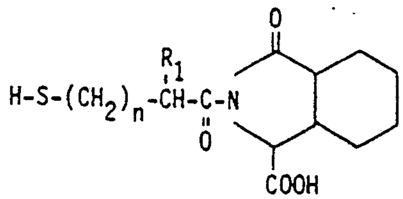

dans laquelle R, $R_1$ et n sont tels que définis dans la revendication 1, avec un agent d'acylation présentant la formule:

$$R_3—CO—X'$$

dans laquelle:

$R_3$ est tel que défini dans la revendication 1; et

X' est un groupe partant, en présence d'une base.

6. Un procédé de préparation d'un sel pharmaceutiquement acceptable d'un composé produit par un procédé selon l'une quelconque des revendications 1 à 5, lequel procédé comprend la réaction du composé avec une base ou un acide capable de former un sel pharmaceutiquement acceptable en dérivant.

7. Un procédé selon l'une quelconque des revendications 1 à 6, qui consiste en un procédé de production d'un acide de formule:

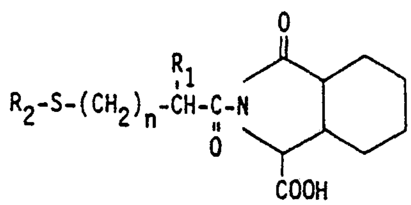

dans laquelle:

$R_1$ représente un atome d'hydrogène ou un radical alkyle inférieur contenant 1 à 3 atomes de carbone;

$R_2$ représente un atome d'hydrogène ou $R_3$—CO— (dans lequel $R_3$ représente un radical alkyle inférieur, furyle, benzo(b)furyle, thiényle, benzo(b)thiényle, pyridyle, quinolyle, isoquinolyle); et

n est égal à 0 ou 1, ou un de leurs sels pharmaceutiquement acceptables.

8. Un procédé selon la revendication 7, qui consiste en un procédé de préparation d'un acide dans lequel $R_1$ est un atome d'hydrogène ou un radical méthyle et $R_2$ est un atome d'hydrogène ou $R_3$—CO— (dans lequel $R_3$ est un radical méthyle ou phényle), ou un de leurs sels basiques pharmaceutiquement acceptables en dérivant.

9. Un procédé selon l'une quelconque des revendications 1 à 6, consistant en un procédé de préparation d'acide octahydro-2-(3-mercapto-propanoyl)-3-oxo-1H-isoindole-1-carboxylique ou d'un de ses sels pharmaceutiquement acceptables, ou d'acide octahydro-2-(3-mercapto-2-méthyl-propanoyl)-3-oxo-1H-isoindole-1-carboxylique ou d'un de ses sels pharmaceutiquement acceptables en dérivant.

**Patentansprüche für die Vertragsstaaten: BE CH DE FR GB IT LI LU NL SE**

1. Octahydro-2-(φ-mercaptoalkanoyl)-3-oxo-1H-isoindol-1-carbonsäure oder -ester mit der Formel

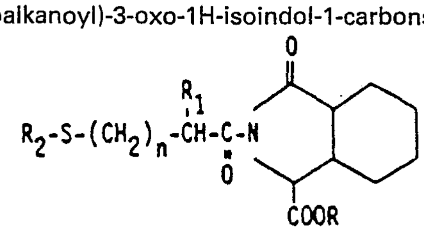

worin R Wasserstoff oder ein Niederalkyl bedeutet, $R_1$ Wasserstoff, Niederalkyl oder Benzyl bedeutet, $R_2$

Wasserstoff oder $R_3$—CO— (worin $R_3$ für Niedrigalkyl, Furyl, Benzo(b)furyl, Thienyl, Benzo(b)thienyl, Pyridyl, Chinolyl, Isochinolyl, Phenyl oder substituiertes Phenyl mit einem oder zwei Substituenten, ausgewählt aus Fluor, Chlor, Brom, Niedrigalkyl und Niedrigalkoxy, steht) bedeutet und n 0 oder 1 bedeutet, und worin Niedrigalkyl und Niedrigalkoxy 1—4 Kohlenstoffatome enthaltende gerade oder verzweigte Gruppen umfassen, oder ein pharmazeutisch akzeptables Salz davon, wenn R Wasserstoff und/oder wenn $R_3$ Pyridyl, Chinolyl oder Isochinolyl bedeutet.

2. Säure nach Anspruch 1 mit der Formel

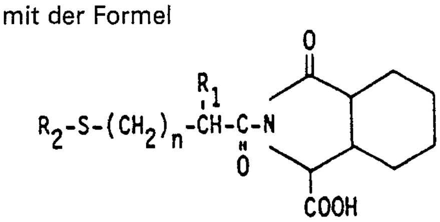

worin $R_1$ Wasserstoff oder 1—3 Kohlenstoffatome enthaltendes Niedrigalkyl bedeutet, $R_2$ Wasserstoff oder $R_3$—CO— (worin $R_3$ für 1—3 Kohlenstoffatome enthaltendes Niedrigalkyl, Phenyl, Furyl, Benzo(b)furyl, Thienyl, Benzo(b)thienyl, Pyridyl, Chinolyl oder Isochinolyl steht) bedeutet, und n 0 oder 1 bedeutet, oder ein pharmazeutisch akzeptables Salz davon.

3. Säure nach Anspruch 2, worin $R_1$ Wasserstoff oder Methyl bedeutet und $R_2$ Wasserstoff oder $R_3$—CO— (worin $R_3$ für Methyl oder Phenyl steht) bedeutet, oder ein pharmazeutisch akzeptables basisches Salz davon.

4. Octahydro-2-(3-mercaptopropanoyl)-3-oxo-1H-isoindol-1-carbonsäure oder ein pharmazeutisch akzeptables Salz davon.

5. Octahydro-2-(3-mercapto-2-methylpropanoyl)-3-oxo-1H-isoindol-1-carbonsäure oder ein pharmazeutisch akzeptables Salz davon.

6. Verfahren zur Herstellung einer Octahydro-2-(ω-mercaptoalkanoyl)-1H-isoindol-1-carbonsäure nach Anspruch 1, welches die Acylierung eines Niedrigalkylesters oder Trimethylsilylesters einer Octahydro-1H-isoindol-1-carbonsäure mit einem Säurehalogenid der Formel

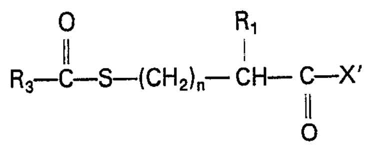

worin $R_1$, $R_3$ und n der in Anspruch 1 angeführten Definition entsprechen und X' für Chlor oder Brom steht, und die Hydrolyse mit einer Base zum Erhalt der Säure nach Anspruch 1, worin R und $R_2$ Wasserstoff bedeuten, umfaßt.

7. Pharmazeutische Zusammensetzung umfassend eine Octahydro-2-(ω-mercaptoalkanoyl)-1H-isoindol-1-carbonsäure oder -ester nach einem der Ansprüche 1 bis 5, oder ein pharmazeutisch akzeptables Salz davon nach einem der Ansprüche 1 bis 5, und einen pharmazeutisch akzeptablen Träger.

**Patentansprüche für den Vertragsstaat: AT**

1. Verfahren zur Herstellung einer Octahydro-2-(ω-mercaptoalkanoyl)-3-oxo-1H-isoindol-1-carbonsäure oder -esters mit der Formel

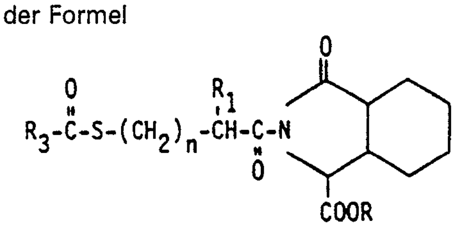

worin R Wasserstoff oder Niedrigalkyl bedeutet, $R_1$ Wasserstoff, Niedrigalkyl oder Benzyl bedeutet, $R_3$ Niedrigalkyl, Furyl, Benzo(b)furyl, Thienyl, Benzo(b)thienyl, Pyridyl, Chinolyl, Isochinolyl, Phenyl oder substituiertes Phenyl mit einem oder zwei Substituenten, ausgewählt aus Fluor, Chlor, Brom, Niedrigalkyl und Niedrigalkoxy, bedeutet, und n 0 der 1 bedeutet, und worin Niedrigalkyl und Niedrigalkoxy 1—4 Kohlenstoffatome enthaltende gerade oder verzweigte Gruppen umfassen; welches Verfahren die Umsetzung einer Octahydro-1H-isoindol-1-carbonsäure oder -ester mit der Formel

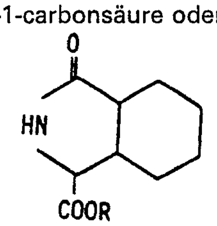

worin R die zuvor angegebene Bedeutung hat, mit einem Säurehalogenid der Formel

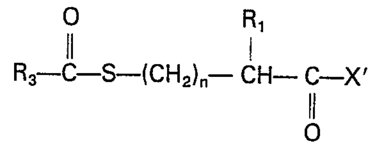

worin X' Halogen bedeutet und $R_1$, $R_3$ und n die zuvor angegebene Bedeutung haben, in einem basischen Medium umfaßt.

2. Verfahren zur Herstellung einer Octahydro-2-(ω-mercaptoalkanoyl)-3-oxo-1H-isoindol-1-carbonsäure mit der Formel

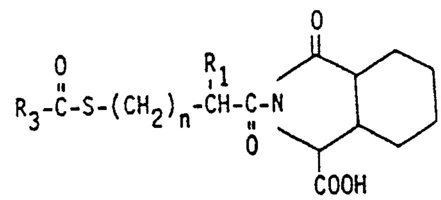

worin $R_1$, $R_3$ und n die in Anspruch 1 angegebene Bedeutung haben, welches Verfahren (1) die Umsetzung eines Trialkylsilylesters von Octahydro-1H-isoindol-1-carbonsäure mit einem Säurehalogenid der Formel

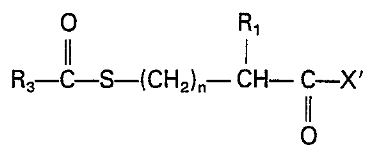

worin $R_1$, $R_3$, n und X' der in Anspruch 1 angegebenen Bedeutung entsprechen, und (2) die Hydrolyse des als Zwischenprodukt erzeugten Trialkylsilylesters zur Bildung der freien Säure umfaßt.

3. Verfahren zur Herstellung einer Octahydro-2-(ω-mercaptoalkanoyl)-3-oxo-1H-isoindol-1-carbonsäure mit der Formel

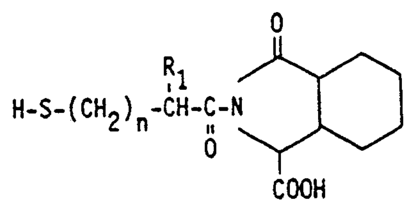

worin $R_1$, n und X die in Anspruch 1 angegebene Bedeutung haben, welches Verfahren die Hydrolyse einer Octahydro-2-(ω-mercaptoalkanoyl)-3-oxo-1H-isoindol-1-carbonsäure oder -esters mit der Formel

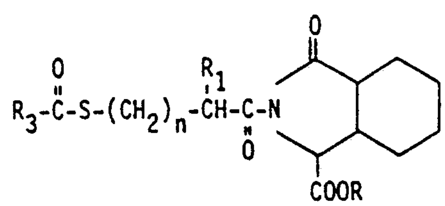

worin R, $R_1$, $R_3$ und n die in Anspruch 1 angegebene Bedeutung haben, umfaßt.

4. Verfahren zur Herstellung einer Octahydro-2-(ω-mercaptoalkanoyl)-3-oxo-1H-isoindol-1-carbonsäure mit der Formel

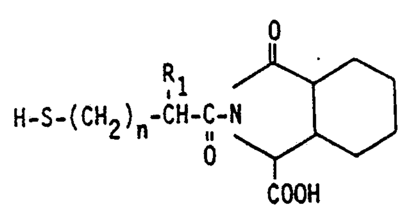

worin $R_1$ und n die in Anspruch 1 angegebene Bedeutung haben, welches Verfahren die Ammonolyse einer Octahydro-2-(ω-mercaptoalkanoyl)-3-oxo-1H-isoindol-1-carbonsäure mit der Formel

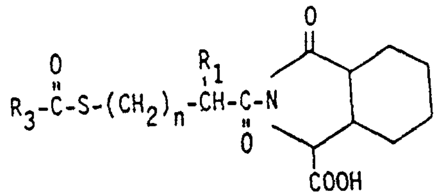

worin $R_1$, $R_3$ und n die in Anspruch 1 angegebene Bedeutung haben, umfaßt.

5. Verfahren zur Herstellung einer Octahydro-2-(ω-mercaptoalkanoyl)-3-oxo-1H-isoindol-1-carbonsäure oder -esters mit der Formel

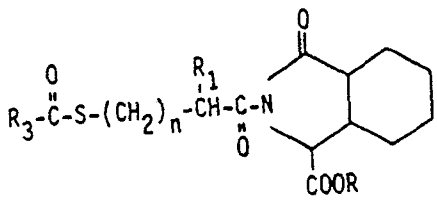

worin R, $R_1$, $R_3$ und n die in Anspruch 1 angegebene Bedeutung haben, welches Verfahren die Umsetzung einer Octahydro-2-(ω-mercaptoalkanoyl)-3-oxo-1H-isoindol-1-carbonsäure oder -esters mit der Formel

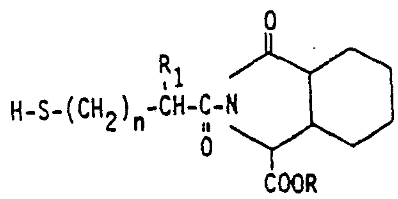

worin R, $R_1$ und n die in Anspruch 1 angegebene Bedeutung haben, mit einem Acylierungsmittel mit der Formel

$$R_3—CO—X'$$

worin $R_3$ die in Anspruch 1 angegebene Bedeutung hat und X' eine austretende Gruppe ist in Anwesenheit einer Base umfaßt.

6. Verfahren zur Herstellung eines pharmazeutisch akzeptablen Salzes einer mittels eines Verfahren nach einem der Ansprüche 1 bis 5 hergestellten Verbindung, welches Verfahren die Umsetzung der Verbindung mit einer Base oder einer Säure, die ein pharmazeutisch akzeptables Salz bilden kann, umfaßt.

7. Verfahren nach einem der Ansprüche 1 bis 6, welches ein Verfahren zur Herstellung einer Säure mit der Formel

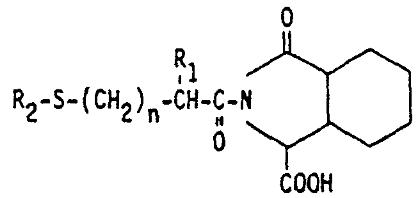

worin $R_1$ Wasserstoff oder 1—3 Kohlenstoffatome enthaltendes Niedrigalkyl bedeutet, $R_2$ Wasserstoff oder $R_3$—CO— (worin $R_3$ für 1—3 Kohlenstoffatome enthaltendes Niedrigalkyl, Phenyl, Furyl, Benzo(b)furyl, Thienyl, Benzo(b)thienyl, Pyridyl, Chinolyl oder Isochinolyl steht) bedeutet und n 0 oder 1 bedeutet, oder eines pharmazeutish akzeptablen Salzes davon ist.

8. Verfahren nach Anspruch 7, welches ein Verfahren zur Herstellung einer Säure, worin $R_1$ Wasserstoff oder Methyl bedeutet und $R_2$ Wasserstoff oder $R_3$—CO— (worin $R_3$ für Methyl oder Phenyl steht), bedeutet, oder eines pharmazeutisch akzeptablen basischen Salzes davon ist.

9. Verfahren nach einem der Ansprüche 1 bis 6, welches ein Verfahren zur Herstellung von Octahydro-2-(3-mercaptopropanolyl)-3-oxo-1H-isoindol-1-carbonsäure oder eines pharmazeutisch akzeptablen Salzes davon; oder von Octahydro-2-(3-mercapto-2-methylpropanoyl)-3-oxo-1H-isoindol-1-carbonsäure oder eines pharmazeutisch akzeptablen Salzes davon ist.